# EUROPÄISCHE PATENTSCHRIFT

(11) **EP 3 665 465 B1**
(45) Veröffentlichungstag und Bekanntmachung des Hinweises auf die Patenterteilung: **26.02.2025**
(21) Anmeldenummer: 18749831.6
(22) Anmeldetag: 10.08.2018
(51) Int. Cl.: G01N 1/34, B01D 61/24, B01D 61/28, B01D 63/08, G01N 30/14

(54) **DIALYSEZELLE ZUR PROBENVORBEREITUNG FÜR DIE IONENCHROMATOGRAPHIE**
DIALYSIS CELL FOR SAMPLE PREPARATION FOR ION CHROMATOGRAHY
CELLULE DE DIALYSE PERMETTANT LA PRÉPARATION D'ÉPROUVETTES POUR CHROMATOGRAPHIE À ÉCHANGE D'IONS

(30) Priorität: 11.08.2017 EP 17185845
(43) Veröffentlichungstag der Anmeldung: 17.06.2020
(73) Patentinhaber: METROHM AG, 9100 Herisau (CH)
(72) Erfinder: LÄUBLI, Markus, 9100 Herisau (CH); FRENZEL, Wolfgang, 10823 Berlin (DE); MARKEVICIUTE, Inga, 12353 Berlin (DE); ABDERHALDEN, Daniel, 9205 Waldkirch (CH)
(74) Vertreter: Hepp Wenger Ryffel AG
(86) Internationale Anmeldenummer: PCT/EP2018/071821
(87) Internationale Veröffentlichungsnummer: WO 2019/030404

(56) Entgegenhaltungen:
- EP-A1- 0 820 804
- EP-A2- 0 180 321
- WO-A1-2005/119245
- US-A- 4 311 789
- US-A- 4 491 011
- US-A- 4 837 157
- US-A1- 2005 191 759
- US-A1- 2016 056 030
- DE BORBA B M ET AL: "On-line dialysis as a sample preparation technique for ion chromatography", JOURNAL OF CHROMATOGRAP, ELSEVIER, AMSTERDAM, NL, vol. 919, no. 1, 1 June 2001 (2001-06-01), pages 59 - 65, XP004247989, ISSN: 0021-9673, DOI: 10.1016/S0021-9673(01)00797-X

## Beschreibung

Die vorliegende Erfindung betrifft eine Dialysezelle zur Probenvorbereitung gemäss dem Oberbegriff von Anspruch 1, eine Vorrichtung zur Probenvorbereitung gemäss dem Oberbegriff von Anspruch 7 sowie eine Verwendung einer Dialysezelle gemäss Anspruch 10. Die vorliegende Erfindung betrifft weiter ein Ionenchromatographiesystem und/oder ein System für High Performance Liquid Chromatography umfassend eine Dialysezelle gemäss dem Oberbegriff von Anspruch 1.

Bei vielen chemischen Analyseverfahren ist es notwendig, eine Probe einer Vorbereitung zu unterziehen, bevor eine eigentliche Analyse durchgeführt werden kann. So enthalten beispielsweise Proben für die Analyse mittels Ionenchromatographie neben den zu analysierenden Ionen, dem oder den Analyten, häufig Matrixbestandteile, die korrosiv sind oder zu Ausfällungen führen können. Diese machen die Analyse mitunter schwierig oder gar unmöglich. Stark belastete Proben können darüber hinaus die Trennsäule beschädigen oder zu einer signifikanten Reduktion ihrer Lebensdauer führen. Zahlreiche Analytikanwendungen setzen eine geeignete Probenvorbereitung entsprechend zwingend voraus. Während traditionell sämtliche Probenvorbereitungsschritte manuell durchgeführt wurden, haben sich in den letzten Jahren sog. Inline-Probenvorbereitungstechniken durchgesetzt. Diese ermöglichen, das Vorbereitungsverfahren vollständig zu automatisieren.

So beschreibt die EP 0 820 804 A1 eine Inline-Dialyse, welche insbesondere für die Ionenchromatographie zur Anwendung kommt. Das besagte Dialyseverfahren wird mittels einer Dialysezelle durchgeführt, die einen Donorkanal sowie einen parallel dazu verlaufenden Akzeptorkanal aufweist. Zwischen diesen beiden Kanälen ist eine selektivpermeable Dialysemembran angeordnet. Die Dialyse wird als sog. Stopped-Flow-Dialyse durchgeführt. Dabei wird eine flüssige Probe, auch Donorlösung genannt, kontinuierlich durch den Donorkanal geleitet. Ein Flüssigkeitsstrom einer Akzeptorlösung durch den Akzeptorkanal wird zeitgleich für eine gewisse Dauer gestoppt. Durch den Konzentrationsgradienten zwischen der Donor- und der Akzeptorlösung kommt es zu einer Diffusion der Analyten von der Donorlösung in die Akzeptorlösung. Nachdem sich im Wesentlichen ein Konzentrationsgleichgewicht eingestellt hat, wird die Akzeptorlösung einer ionenchromatographischen Trennung zugeführt.

Die Zeitspanne vom Anhalten des Akzeptorstroms bis zum Ende der Dialyse, welches typischerweise durch ein Weiterleiten der Akzeptorlösung aus der Dialysezelle gegebenen ist, wird als Dialysezeit *t_{D}* bezeichnet.

Die Wiederfindungsrate R wird als Verhältnis der Analytenkonzentration im Akzeptorkanal zur Analytenkonzentration im Donorkanal zum Ende der Dialysezeit *t_{D}* definiert. Die Wiederfindungsrate R steigt typischerweise mit zunehmender Dialysezeit *t_{D}* streng monoton an.

Als Äquilibrationszeit *t_{A}* wird die Zeit bezeichnet, in der die Analytenkonzentration im Akzeptorkanal im Wesentlichen jene im Donorkanal erreicht hat. Im vorliegenden Zusammenhang entspricht die Äquilibrationszeit *t_{A}* derjenigen Dauer der Dialyse, nach welcher die relative Änderung der Analytenkonzentration im Akzeptorkanal erstmals weniger als 2%/min beträgt.

Die beschriebene Technik trennt nicht nur Partikel von dem oder den Analyten, sondern auch Kolloide, Ölbestandteile und grosse Moleküle. Insbesondere proteinhaltige Proben können damit direkt mittels Ionenchromatographie verarbeitet werden. Dies erspart zeitaufwändige manuelle Arbeitsschritte, wie beispielsweise eine Ausfällung der Proteine mittels Carrez-Reagens. Auch wenn Proben mit Partikeln belastet sind und eine Filtration aufgrund von blockierenden Filtern nicht angewendet werden kann, stellt die Inline-Dialyse eine praktikable Probenvorbereitung dar.

Obwohl diese Probenvorbereitungstechnik viele Vorteile hat, besteht in der Regel das Problem, dass die Dialyse der geschwindigkeitslimitierende Schritt des Analyseprozesses ist. Dies ist insbesondere bei einer seriellen Verarbeitung vieler Proben von Nachteil, da das Verfahren durch die Probenvorbereitung zeit- und kostenintensiv wird. Darüber hinaus besteht das Problem, dass es bei stark belasteten Proben zu einem teilweisen Durchbruch von Matrixverunreinigungen durch die Dialysemembran kommen kann. Ein weiterer Nachteil stellt der vergleichsweise hohe Probenaufwand bei der Stopped-Flow-Dialyse dar.

US 4`311`789 und US 4'491'011 offenbaren Analysegeräte, denen eine Dialyseeinheit vorgeschaltet ist. Die Dialysezelle ist jeweils so ausgestaltet, dass ein komplexes Fluid-Medium, namentlich Blut, durch einen Kanal geführt wird, wobei sich in diesem Kanal eine weitere Fluidführung befindet, die vollständig oder teilweise gebildet ist aus einer selektivpermeablen Membran, beispielsweise eine Faser oder Kapillare aus einer selektivpermeablen Membran. In dieser weiteren Fluidführung wird die Akzeptorlösung bereitgestellt. Wenn das Volumen des Akzeptorkanals zumindest abschnittsweise ein Volumen *V_{A}* hat, das kleiner ist als ein parallel dazu verlaufendes Volumen *V_{D}* des Donorkanals, kann dies vorteilhafte Auswirkungen auf die Äquilibrationszeit haben. Die bekannten Dialysezellen operieren jedoch ohne geeignete Kontrolle der Druckdifferenz zwischen Donorkanal und Akzeptorkanal. Durch Druckgradienten im Donor- und/oder Akzeptorkanal kommt zu unerwünschter Filtration. Durch den Aufbau eines Filterkuchens insbesondere bei partikelbeladenen Matrizen ändert sich die Durchlässigkeit der Dialysemembran. Die Durchlässigkeit kann sich sogar für die individuellen Analyten jeweils unterschiedlich verändern. Die Äquilibrationszeit *t_{A}* wird dadurch in unvorhersehbarer Weise beeinflusst und schwer kontrollierbar.

Es ist daher eine Aufgabe der vorliegenden Erfindung, die oben genannten Nachteile im Stand der Technik zu überwinden. Insbesondere ist es eine Aufgabe der vorliegenden Erfindung, die Äquilibrationszeit *t_{A}* zu verkürzen und damit einen insgesamt höheren Probendurchsatz zu erzielen. Darüber hinaus steht der vorliegenden Erfindung das Problem zu Grunde, bei stark belasteten Proben eine bessere Abtrennung des oder der Analyten von der Matrix zu erzielen. Zudem ist es eine Aufgabe der vorliegenden Erfindung, eine Vorrichtung und ein Verfahren zur Probenvorbereitung durch Dialyse bereitzustellen, durch welche eine zuverlässige und präzise Äquilibration in einer kontrollierbaren Äquilibrationszeit *t_{A}* ermöglicht wird.

Diese Aufgaben werden durch eine Dialysezelle zur Probenvorbereitung für die Ionenchromatographie, gelöst, welche die Merkmale in Anspruch 1 aufweist. Die Dialysezelle umfasst einen Donorkanal und einen parallel dazu verlaufenden Akzeptorkanal sowie eine selektivpermeable Dialysemembran. Der Donorkanal und der Akzeptorkanal sind durch die selektivpermeable Dialysemembran voneinander getrennt. Dadurch kann insbesondere ein in einer Donorlösung im Donorkanal gelöster Analyt durch die Dialysemembran in die Akzeptorlösung im Akzeptorkanal gelangen. Der Akzeptorkanal hat zumindest abschnittsweise ein Volumen *V_{A}*, das kleiner ist als ein parallel dazu verlaufendes Volumen *V_{D}* des Donorkanals.

Es hat sich gezeigt, dass durch einen derartigen asymmetrischen Aufbau der Dialysezelle die Äquilibrationszeiten *t_{A}* markant gesenkt werden können. Dies führt zu kürzeren Dialysezeiten *t_{D}* und damit zu einem insgesamt höheren Probendurchsatz, was mit einer deutlichen Zeit- und Kostenersparnis verbunden ist. Darüber hinaus wird durch kürzere Dialysezeiten *t_{D}* der Zeitraum, während dem die Dialysemembran mit der Probenmatrix in Kontakt ist, reduziert. Dadurch kann der Durchbruch von Matrixverunreinigungen signifikant reduziert werden. Zudem kann die Dialyse mit einem deutlich geringeren Probenvolumen durchgeführt werden.

Die erfindungsgemässe Dialysezelle umfasst zwei Halbzellen, zwischen denen die selektivpermeable Dialysemembran angeordnet ist. Der Donorkanal und der Akzeptorkanal sind als jeweils eine Vertiefung in einer Kontaktfläche einer der Halbzellen mit der Dialysemembran ausgebildet. Dieser Aufbau der Dialysezelle hat sich als besonders robust, kostengünstig in der Fertigung und benutzerfreundlich im Hinblick auf Betrieb und Wartung erwiesen.

Überdies sind bei einem solchen Aufbau die Volumina beider Halbzellen bekannt und konstant. Es kann durch geeignete Wahl der Menge/Flussrate der zugeführten und abgeführten Donor- respektive Akzeptorflüssigkeit ein Überdruck oder Unterdruck in einer der Halbzellen vermieden werden. Ein solcher Überdruck oder Unterdruck führt zu Filtration, die sich störend auf das sich zufolge Diffusion einstellende Konzentrationsgleichgewicht der Analyten in der Donor- und Akzeptorlösung auswirken kann. Beispielsweise kann bei einem Überdruck im Donorkanal Filtrat in den Akzeptorkanal übertreten.

Die druckfreie Dialyse, das heisst die Dialyse in Abwesenheit eines Druckgradienten innerhalb der Dialysezelle, erweist sich insbesondere dann als Vorteil, wenn Donorflüssigkeiten der Dialyse unterzogen werden sollen, die auf wechselnden Medien oder Matrizen basieren. Bei gleichbleibender Matrix, wie sie beispielsweise bei der Dialyse und/oder Analyse von Blutserum in medizinischen Anwendungen vorliegt, lassen sich die Flussrate und damit der Druck gestützt auf Erfahrungswerte mit entsprechendem Aufwand zweckmässig einstellen, ohne dass dadurch die Präzision der Analysewerte übermässig beeinträchtigt würde. Bei wechselnden Matrizen, wie sie dagegen in der Ionenanalytik vielfach auftreten, führt eine Druckdifferenz zu unkontrollierbaren und unvorhersehbaren Dialyszeiten oder Aequilibrationszeiten.

Die Druckkontrolle wird zusätzlich unterstützt, wenn die Dialyse im Stopped-Flow Verfahren durchgeführt wird.

In einer bevorzugten Ausführungsform der vorliegenden Erfindung hat der Akzeptorkanal auf wenigstens 50%, vorzugsweise wenigstens 70%, bevorzugterweise wenigstens 90%, seiner Länge ein Volumen *V_{A}*, das kleiner ist als ein parallel dazu verlaufendes Volumen *V_{D}* des Donorkanals. In einer weiter bevorzugten Ausführungsform der vorliegenden Erfindung hat der Akzeptorkanal auf seiner gesamten Länge ein Volumen *V_{A}*, das kleiner ist als ein parallel dazu verlaufendes Volumen *V_{D}* des Donorkanals.

Der Akzeptorkanal kann zumindest abschnittsweise ein Volumen pro Längeneinheit *V_{A}*/*L* von 0.005 mm³/mm bis 2.0 mm³/mm, vorzugsweise von 0.020 mm³/mm bis 1.5 mm³/mm, bevorzugterweise von 0.10 mm³/mm bis 1.0 mm³/mm, haben. Der Donorkanal kann zumindest abschnittsweise ein Volumen pro Längeneinheit *V_{D}*/*L* von 0.25 mm³/mm bis 3.5 mm³/mm, vorzugsweise von 0.30 mm³/mm bis 3.0 mm³/mm, bevorzugterweise von 0.35 mm³/mm bis 2.0 mm³/mm, haben. Der Donor- und der Akzeptorkanal haben dabei üblicherweise je eine Länge von 10 mm bis 1000 mm, vorzugsweise von 20 mm bis 500 mm, bevorzugterweise von 100 mm bis 300 mm.

Es hat sich gezeigt, dass eine derartige Dimensionierung des Donor- und/oder des Akzeptorkanals insbesondere bei Anwendungen in der Ionenchromatographie vorteilhaft ist. Einerseits können damit deutlich kürzere Äquilibrationszeiten *t_{A}* erzielt werden. Andererseits ist mit diesen Dimensionen sichergestellt, dass die Stoffmenge an Analyt, die der Trennsäule zugeführt wird, innerhalb der Detektionsgrenzen eines üblichen Ionenchromatographiesystems liegt. Im Hinblick auf den Donorkanal ist mit diesen Abmessungen darüber hinaus sichergestellt, dass es auch bei stark, insbesondere mit Partikeln, belasteten Proben nicht zu einem Verstopfen desselben kommt.

Die selektivpermeable Dialysemembran kann eine Porengrösse von 0.01 µm bis 1.0 um, vorzugsweise von 0.02 µm bis 0.5 um, bevorzugterweise von 0.05 µm bis 0.2 um, haben. Da durch den erfindungsgemässen asymmetrischen Aufbau der Dialysezelle mit derselben Dialysemembran eine erforderliche Wiederfindungsrate R schneller erreicht ist, kann für eine gegebene analytische Anwendung auch eine feinporigere Dialysemembran eingesetzt werden, ohne dass eine längere Äquilibrationszeit *t_{A}* in Kauf genommen werden müsste. Dadurch kann insbesondere bei stark belasteten Proben eine bessere Trennung der Matrix von dem oder den Analyten erzielt werden.

Die selektivpermeable Dialysemembran kann aus einem Material bestehen, welches ausgewählt ist aus einer Liste bestehend aus Zelluloseacetat, Zellulosenitrat, Polyvinylidenfluorid, Polycarbonat, Zellulosemischester, Zellulosehydrat und regenerierte Zellulose. Vorzugsweise ist das Material ausgewählt aus einer Liste bestehend aus Polyvinylidenfluorid, Polycarbonat und Zellulosemischester, oder Polyamid. Diese Membranmaterialien haben sich insbesondere bei der Trennung von Metall-Kationen und anorganischen Anionen als besonders vorteilhaft erwiesen.

Der Donorkanal und der Akzeptorkanal können linear, spiralförmig oder mäanderförmig ausgebildet sein. Dies ermöglicht in Relation zur Kanallänge eine besonders kompakte Ausgestaltung der Dialysezelle.

Der Querschnitt durch den Donorkanal und/oder den Akzeptorkanal kann kreissegmentförmig, insbesondere halbkreisförmig, halbellipsenförmig, quadratisch oder rechteckig sein. Diese Kanalgeometrien haben sich sowohl im Hinblick auf die Fertigung der Halbzellen als auch bezüglich ihrer Leistungscharakteristika als besonders vorteilhaft erwiesen.

Besonders bevorzugt ist es jedoch, wenn der Querschnitt des Donorkanals halbkreisförmig ausgebildet ist. Durch die Halbkreisförmige Ausgestaltung können die hochmolekularen Bestandteile, zum Beispiel Fette oder Proteine, ungehindert strömen, bei maximaler Expositionsfläche der Donorlösung zur Dialysemembran hin.

Besonders bevorzugt ist es zudem, wenn der Querschnitt durch den Akzeptorkanal zumindest abschnittweise ein Rechteck ist, welches auf der von der Dialysemembran abgewandten Seite abgerundete Ecken aufweist. Unter abgerundeten Ecken werden hierbei die Abschnitte verstanden, welche den der Dialysmembran gegenüberliegenden Abschnitt des Akzeptorkanals mit den an die Dialysemembran anschliessenden, seitlichen Begrenzungen des Akzeptorkanals verbinden. Diese Abschnitte sind im Querschnitt kreisbogenförmig und weisen einen Krümmungsradius r zwischen 0.05 und 1 mm, bevorzugt zwischen 0.1 und 0.8 mm, ganz besonders bevorzugt zwischen 0.2 und 0.4 mm auf. Zusätzlich oder alternativ ist das Verhältnis der von der Dialysemembran begrenzten Seite zur Tiefe des Rechtecks bevorzugt von 80:1 bis 10:1, besonders bevorzugt von 40:1 bis 15:1 und ganz besonders bevorzugt von 25:1 bis 20:1.

Durch Wahl oben genannter Dimensionen wird der Donorflüssigkeit pro Abschnitt der Zelle ein möglichst kleines Volumen von Akzeptorflüssigkeit gegenübergestellt, bei gleichwohl grosser Kontaktfläche zwischen Akzeptorflüssigkeit und Dialysemembran. Mit anderen Worten hat der Akzeptorkanal ein Volumen *V_{A}*, das minimiert ist im Vergleich zum parallel dazu verlaufenden Volumenabschnitt *V_{D}* des Donorkanals, was die Äquilibrationszeit *t_{A}* verkürzt.

Die abgerundeten Ecken verhindern die Verunreinigung der Zelle durch Ansammlung von Akzeptorflüssigkeit im Vergleich zu nicht-abgerundeten Ecken. Solche Verunreinigung kommt typischerweise durch die verlangsamte Strömungsgeschwindigkeit in der Nähe der Kanalwand zustande, welche in nicht-abgerundeten Ecken akzentuiert würde. Das Problem der Ansammlung von langsam strömender Akzeptorflüssigkeit ergibt sich auch, wenn der Krümmungsradius zu klein gewählt wird. Wird der Krümmungsradius dagegen zu gross gewählt, besteht die Gefahr, dass die Dialysemembran, die in wässriger Lösung auf bis zu 0.15 mm Dicke aufquellen kann, sich an die Wände und/oder den Boden der Vertiefung der Halbzelle anlegt und so die Strömung durch die Halbzelle behindert. Es ist daher bevorzugt, dass der Krümmungsradius zwischen 0.05 und 1 mm, bevorzugt zwischen 0.1 und 0.8 mm, ganz besonders bevorzugt zwischen 0.2 und 0.4 mm beträgt.

Weiter ist es bevorzugt, dass die Dialysezelle so konfiguriert ist, dass im Akzeptorkanal wenigstens ein, bevorzugt mehrere Stützelemente ausgebildet sind, welche die Dialysemembran von der der Dialysemembran abgewandten Seite des Akzeptorkanals beabstanden. Bevorzugt weisen die Stützelemente eine Höhe entsprechend der Tiefe des Akzeptorkanals auf. Bevorzugt sind die Stützelemente zylindersymmetrisch ausgebildet mit einem Stützenoberkantendurchmesser zwischen 5 und 500 um, bevorzugt zwischen 10 und 200 um, ganz besonders bevorzugt zwischen 30 und 60µm. Die Querschnittsgeometrie der Stützen kann aber auch von der Zylindersymmetrie abweichen. Bevorzugt nimmt die Fläche, also die Querschnittsfläche, der Stütze von der membranseitigen Oberkante her ab. Bevorzugt sind die Stützelemente in einem regelmässigen Muster über die Breite des Akzeptorkanals verteilt, besonders bevorzugt ist eine Reihe von Stützelementen in der Mitte des Akzeptorkanals angeordnet. Bevorzugt sind die Abstände zwischen jeweils zwei benachbarten Stützen und/oder zwischen einer Stütze und der direkt benachbarten Kanaloberkante immer gleich.

Es ist eine Funktion des mindestens einen Stützelements, dass das Volumen des Kanals auf der Akzeptorseite konstant gehalten und nicht etwa zufolge Durchhängens der Dialysemembran unter Schwerkraft oder unter Druck verkleinert wird. Grundsätzlich können auch Stützelemente auf der Donorseite angebracht werden, um eine Volumenverkleinerung auf der Donorseite etwa infolge Durchhängens der Dialysemembran unter Schwerkraft oder unter Druck in Richtung Donorkanal zu verhindern. Da der Akzeptorkanal erfindungsgemäss ein kleineres Volumen *V_{A}* im Vergleich zum parallel dazu verlaufenden Volumen *V_{D}* des Donorkanals aufweist, ist der relative Effekt eines Durchhängens im Akzeptorkanal jedoch grösser und eine potentiell daraus resultierende Behinderung der Strömung einschneidender als auf Donorseite.

Die Form und Anzahl der Stützelemente wird so gewählt, dass einerseits die Stabilität der Vorrichtung gewährleistet ist und andererseits die Strömung des Fluids durch den Kanal möglichst wenig behindert wird und des Weiteren möglichst wenig Membranfläche abgedeckt wird. Dafür eignen sich insbesondere Kegelstumpfformen oder mantelseitig konkav gekrümmte Stützelemente.

Bevorzugt werden die Stützelement einstückig mit der Halbzelle ausgebildet. Bevorzugt werden die Stützelemente durch Fräsen, Spritzgiessen, Heissprägen oder 3D-Druck hergestellt.

Die vorliegende Erfindung betrifft zudem eine Vorrichtung zur Probenvorbereitung für ein chemisches Analyseverfahren in einem Dialyseprozess, wobei das chemische Analyseverfahren Chromatographie ist, mit einer Dialysezelle wie oben beschrieben, dadurch gekennzeichnet, dass der Donorkreis eine erste Pumpvorrichtung aufweist, die die Donorflüssigkeit zur Dialysezelle befördert, und eine zweite Pumpvorrichtung aufweist, die die Donorflüssigkeit von der Dialysezelle wegbefördert. Zusätzlich oder alternativ weist der Akzeptorkreis eine erste Pumpvorrichtung auf, die die Akzeptorflüssigkeit zur Dialysezelle befördert, und eine zweite Pumpvorrichtung auf, die die Akzeptorflüssigkeit von der Dialysezelle wegbefördert.

Die jeweils zwei Pumpen pro Fluidkreis können auf gleiche Fördermengen pro Zeiteinheit eingestellt werden und dadurch gleichbleibenden Druck in der jeweiligen Halbzelle, Akzeptor- oder Donorhalbzelle, gewährleisten.

Wenn in Donor- bzw. Akzeptorkreisen nur jeweils eine Pumpe verwendet wird, so wird ein Abschlusselement benötigt, z.B. ein Ventil, welches den Durchfluss stoppt. Damit kann die Stopped-Flow-Bedingung für die Dialyse erreicht werden.

Bevorzugt sind die erste Pumpvorrichtung und die zweite Pumpvorrichtung des Donorkreises in einer Zweikanalpumpe, bevorzugt einer peristaltischen Zweikanalpumpe, kombiniert. Bevorzugt sind zusätzlich oder alternativ die erste Pumpvorrichtung und die zweite Pumpvorrichtung des Akzeptorkreises in einer Zweikanalpumpe, bevorzugt einer peristaltischen Zweikanalpumpe, kombiniert.

Die Verwendung von Zweikanalpumpen haben den Vorteil, dass das der Dialysezelle zugeführte Volumen von Donor- respektive Akzeptorflüssigkeit und das aus der Dialysezelle abgeführte Volumen von Donor- respektive Akzeptorflüssigkeit stets gleich ist, da die gleiche Hubbewegung die Donor- respektive Akzeptorflüssigkeit fördert. Das Zuführen von Flüssigkeit in die jeweilige Halbzelle und das Abführen von Flüssigkeit aus der jeweiligen Halbzelle sind automatisch synchronisiert.

Es ist besonders bevorzugt, dass die erste und die zweite peristaltische Zweikanalpumpe so steuerbar sind, dass sie unabhängig voneinander betrieben werden. Typischerweise wird während der Dialysezeit *t_{D}* ausschliesslich die erste peristaltische Pumpe betrieben, sodass die Donorflüssigkeit kontinuierlich strömt, während die Akzeptorflüssigkeit im Akzeptorkanal gehalten wird. Bevorzugt wird nach Ablauf der Äquilibrationszeit *t_{A}* die erste peristaltische Pumpe gestoppt und ausschliesslich die zweite peristaltische Pumpe betrieben, sodass die Akzeptorflüssigkeit strömt, während die Donorflüssigkeit im Donorkanal gehalten wird.

Eine solche Steuerbarkeit hat den Vorteil, dass ein Unter- respektive Überdruck in einer der Halbzellen verhindert werden kann, der zu unerwünschter Filtration führen könnte.

Bevorzugt wird/werden eine erste Kapillare, welche einen Akzeptorlösungsbehälter, bevorzugt über die zweite Pumpvorrichtung, mit dem Akzeptorkanal verbindet, und/oder eine zweite Kapillare, welche den Akzeptorkanal, bevorzugt über die erste Pumpvorrichtung, mit einer Injektionsvorrichtung für Injektion auf eine Chromatographiesäule, verbindet, so ausgestaltet, dass der Durchmesser der Kapillare maximal 0.5 mm, bevorzugt maximal 0.25 mm aufweist.

Der gewählte Durchmesser soll klein sein, um Diffusion des oder der Analyten während des Transfervorgangs zu beschränken. Wenn die Diffusion entlang der Kapillare zu schnell verläuft, reduziert sich die maximal erreichbare Wiederfindungsrate R, welcher Effekt durch Verwendung von Kapillaren mit kleinem Durchmesser eingedämmt werden soll. Aus demselben Grund wird vorzugsweise auch die Transfergeschwindigkeit klein gehalten, was wiederum durch einen kleinen Kapillarendurchmesser begünstigt wird.

Die vorliegende Erfindung betrifft darüber hinaus die Verwendung einer Dialysezelle wie vorgängig beschrieben zur Probenvorbereitung in einem chemischen Analyseverfahren ausgewählt aus einer Liste bestehend aus Ionenchromatographie (IC) und High Performance Liquid Chromatography (HPLC).

Eine Membran wird als Dialysemembran in einer Dialysezelle wie vorgängig beschrieben zur Probenvorbereitung für ein chemisches Analyseverfahren verwendet, ausgewählt aus einer Liste bestehend aus Ionenchromatographie (IC) und High Performance Liquid Chromatography (HPLC).

Es versteht sich allerdings von selbst, dass im Rahmen der vorliegenden Erfindung auch Kopplungstechniken wie IC-MS, HPLC-MS oder CE-MS zur Anwendung kommen können.

Die beiden oben genannten Verwendungen können die folgenden Schritte umfassen:
- Bereitstellen einer Akzeptorlösung;
- Einleiten einer bestimmten Menge der Akzeptorlösung in den Akzeptorkanal oder in einen den Akzeptorkanal enthaltenden Akzeptorkreis;
- Halten der bestimmten Menge der Akzeptorlösung im Akzeptorkanal oder in den dem Akzeptorkanal enthaltenden Akzeptorkreis;
- Bereitstellen einer zumindest einen Analyten enthaltenden Donorlösung;
- Durchleiten der Donorlösung durch den Donorkanal, wodurch der zumindest eine in der Donorlösung enthaltene Analyt durch die Dialysemembran in die Akzeptorlösung gelangt.

Dabei wird mindestens solange frische Donorlösung durch den Donorkanal geleitet, bis die Konzentration des zumindest einen Analyten in der Akzeptorlösung mindestens 90%, vorzugsweise mindestens 95%, bevorzugterweise mindestens 99%, der Konzentration des zumindest einen Analyten in der Donorlösung beträgt.

Zu dem Zeitpunkt, in dem die Konzentration des Analyten in der Akzeptorlösung den vordefinierten Schwellenwert erreicht, werden sodann die folgenden Schritte ausgeführt:
- Halten der Donorlösung im Donorkanal (2) oder in einem den Donorkanal enthaltenden Donorkreis;
- Ableiten der Akzeptorlösung und Zuführen der Akzeptorlösung zu einer Analysevorrichtung, insbesondere ausgewählt aus einer Liste bestehend aus einer Ionenchromatographievorrichtung (IC), einer Vorrichtung für High Performance Liquid Chromatography (HPLC), einer Kapillarelektrophoresevorrichtung (CE) und einer Massenspektrometrievorrichtung (MS) .

Das Halten der Donorlösung im Donorkanal (2) oder in einem den Donorkanal enthaltenden Donorkreis während des Ableitens der Akzeptorlösung hat den Vorteil, dass auch während des Transfers der Akzeptorlösung zur Analysevorrichtung es zu keinen Druckdifferenzen zwischen dem Donor- und dem Akzeptorkanal kommt, etwa zufolge nicht-synchroner Schaltung der jeweils am Donor- bzw. Akzeptorkreis befindlichen Pumpen. Dadurch wird gewährleistet, dass es nicht zu unerwünschter Filtration kommt, welche das eingestellte Gleichgewicht verfälschen könnte.

Frische Donorlösung kann dabei kontinuierlich durch den Donorkanal geleitet werden. Die Flussrate der Donorlösung durch den Donorkanal kann in einem Bereich von 0.01 ml/min bis 10.0 ml/min, vorzugsweise von 0.05 ml/min bis 5.0 ml/min, bevorzugt von 0.10 ml/min bis 1.0 ml/min, liegen. Diese Flussraten haben sich im Hinblick auf die üblicherweise beobachtete Geschwindigkeit des Massenaustausches bei einer derartigen Dialyse bewährt.

Die vorliegende Erfindung betrifft darüber hinaus ein Ionenchromatographiesystem (IC) und/oder ein System für High Performance Liquid Chromatography (HPLC) umfassend eine Dialysezelle wie oben beschrieben.

Weitere Vorteile und Einzelmerkmale der Erfindung ergeben sich aus der nachfolgenden Beschreibung eines Ausführungsbeispiels und aus den Zeichnungen.

Es zeigen schematisch:
- Figur 1:: Perspektivische Explosionsdarstellung einer erfindungsgemässen Dialysezelle;
- Figur 2:: Querschnittsdarstellung einer Dialysezelle aus dem Stand der Technik;
- Figur 3:: Querschnittsdarstellung einer erfindungsgemässen Dialysezelle;
- Figur 4:: Ionenchromatographiesystem (IC) umfassend eine erfindungsgemässe Dialysezelle;
- Figur 5:: Einfluss der Volumens *V_{A}* der Akzeptorkanals auf die Wiederfindungsrate R;
- Figuren 6 und 7:: Vergleich einer erfindungsgemässen Dialysezelle mit einer solchen aus dem Stand der Technik hinsichtlich des Durchbruchs von Ligninen.
- Figuren 8 und 9:: Beispiel einer Probenaufbereitung mit einer erfindungsgemässen Dialysezelle.
- Figuren 10 und 11:: Querschnitte durch einen Akzeptorkanal einer erfindungsgemässen Dialysezelle mit und ohne Stützelemente.
- Figur 12:: Perspektivische Ansicht eines Akzeptorkanals einer erfindungsgemässen Dialysezelle.
- Figuren 13 und 14:: Querschnitte durch zwei alternative Anordnungen von zwei Halbzellen einer erfindungsgemässen Dialysezelle, jeweils mit Stützelementen.
- Figur 15:: Schematische Darstellung einer Vorrichtung zur Probenvorbereitung für ein chemisches Analyseverfahren in einem Dialyseprozess.

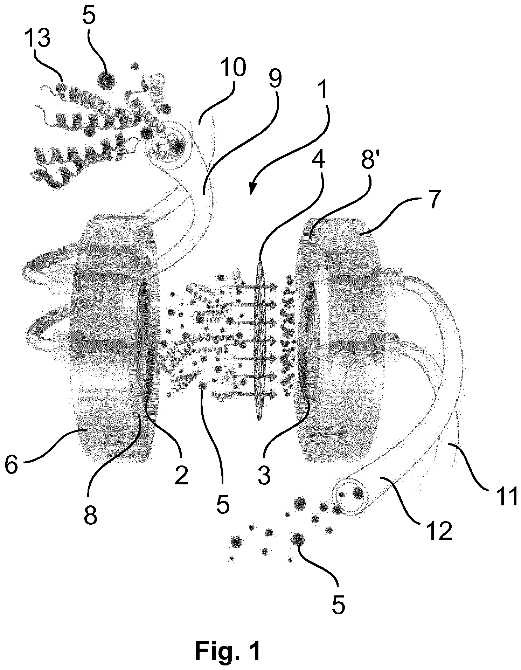

Wie aus Figur 1 ersichtlich ist, besteht eine Ausführungsform einer erfindungsgemässen Dialysezelle 1 aus zwei Halbzellen 6 und 7, zwischen denen eine selektivpermeable Dialysemembran 4 angeordnet ist. Die Halbzellen 6 und 7 weisen an ihren Kontaktflächen 8, 8' mit der Dialysemembran 4 jeweils eine spiralförmige Vertiefung auf, welche den Donorkanal 2 bzw. den Akzeptorkanal 3 bildet. Der Donorkanal 2 ist mit einer Zuleitung 9 verbunden, über welchen eine Probe zugeleitet werden kann. Darüber hinaus ist der Donorkanal 2 mit einer Ableitung 10 verbunden, mit welchem die Probe nach Durchlaufen der Dialysezelle 1 abgeleitet und üblicherweise einer Entsorgung 17 zugeführt werden kann. Auch der Akzeptorkanal 3 ist mit einer Zuleitung 11 und einer Ableitung 12 verbunden. Die Zuleitung 11 und die Ableitung 12 des Akzeptorkanals 3 können zu einem Akzeptorkreis zusammengeschlossen werden. Die durch den Donorkanal 2 geleitete Probenlösung enthält zumindest einen Analyten, der hier symbolisch als Metallion 5 dargestellt ist, sowie Matrixmoleküle, die vorliegend symbolisch als Proteine 13 dargestellt sind. Die Metallionen 5 können die selektivpermeable Dialysemembran 4 durchqueren (vgl. Pfeile), während die Proteine 13 zurückgehalten werden. Damit kann eine Metallionen 5 enthaltende Probe, beispielsweise für die Ionenchromatographie, aufbereitet werden.

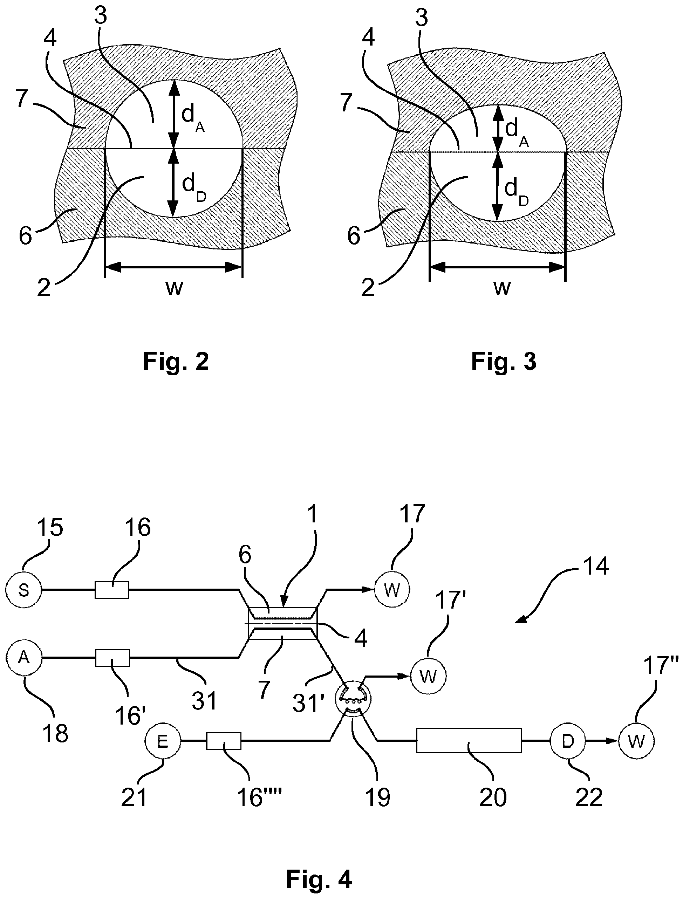

Die Figur 2 zeigt einen Querschnitt durch eine gattungsgemässe Dialysezelle aus dem Stand der Technik. Es ist zu erkennen, dass der Donorkanal 2 sowie der Akzeptorkanal 3 eine identische Breite w haben. Zudem ist auch die Tiefe *d_{D}* des Donorkanals gleich der Tiefe *d_{A}* des Akzeptorkanals. In der Figur 3 ist hingegen ein der Figur 2 entsprechender Querschnitt einer erfindungsgemässe Dialysezelle abgebildet. Sowohl der Donorkanal 2 als auch der Akzeptorkanal 2 weisen nach wie vor die Breite w auf. Auch ist die Tiefe des Donorkanals *d_{D}* gegenüber dem Beispiel gemäss Figur 2 identisch. Allerdings weist der Akzeptorkanal eine reduzierte Tiefe *d_{A}* auf. Es versteht sich von selbst, dass der Akzeptorkanal 3 damit im Bereich der Schnittebene ein Volumen pro Längeneinheit *V_{A}*/*L* aufweist, das kleiner ist als ein entsprechendes Volumen pro Längeneinheit *V_{D}*/*L* des Donorkanals 2.

Die Figur 4 zeigt ein Chromatographiesystem 14 umfassend eine erfindungsgemässe Dialysezelle 1. Im besagten System 14 ist eine Probenlösung, welche vorliegend auch als Donorlösung bezeichnet wird, in einem Probenbehälter 15 bereitgestellt. Die Donorlösung wird mittels einer Pumpe 16 durch die erste Halbzelle 6 der Dialysezelle 1 gepumpt und danach in einem Sammelbehälter 17 zwecks Entsorgung gesammelt. Eine Akzeptorlösung ist in einem Akzeptorbehälter 18 bereitgestellt und wird mittels einer weiteren Pumpe 16' durch die zweite Halbzelle 7 der Dialysezelle 1 gepumpt. Überschüssige Akzeptorlösung wird ebenfalls in einem Sammelbehälter 17` zwecks Entsorgung gesammelt. Wie vorgängig bereits erläutert wurde, wird zur Durchführung einer sogenannten Stopped-Flow-Dialyse der Strom der Akzeptorlösung, im Vorliegenden auch als Akzeptorstrom bezeichnet, durch die Halbzelle 7 gestoppt, während der Strom der Donorlösung, im Vorliegenden auch als Donorstrom bezeichnet, durch die Halbzelle 6 weitergeführt wird. Dies wird solange aufrechterhalten, bis die Akzeptorlösung innerhalb der Halbzelle 7 einen gewünschten Mindestanteil der Konzentration des Analyten der Donorlösung innerhalb der Halbzelle 6 aufweist. Ein solcher Mindestanteil kann beispielsweise 90%, 95% oder 99% sein.

Nach erfolgter Dialyse kann das Injektionsventil 19 umgeschaltet werden, wodurch der Analyt der Chromatographiesäule 20 zugeführt wird. Während der Analyt der Chromatographiesäule 20 zugeführt wird, wird der Donorfluss gestoppt. Der eigentliche Chromatographieteil des Chromatographiesystems 14 ist vorliegend stark vereinfacht dargestellt. Ein Eluent ist in einem Eluentenbehälter 21 bereitgestellt und wird mittels einer Pumpe 16ʺʺ, insbesondere einer Hochdruckpumpe, über das Injektionsventil 19 durch die Trennsäule 20 gepumpt. Nach erfolgter Detektion mittels des Detektors 22 wird die ionenchromatographisch getrennte Probe ebenfalls in einem Sammelbehälter 17" zwecks Entsorgung gesammelt. Es versteht sich allerdings von selbst, dass im Rahmen der vorliegenden Erfindung auch sog. Tandemtechniken, beispielsweise eine Kopplung eines Leitfähigkeitsdetektors und eines Massenspektrometers (MS), realisierbar sind.

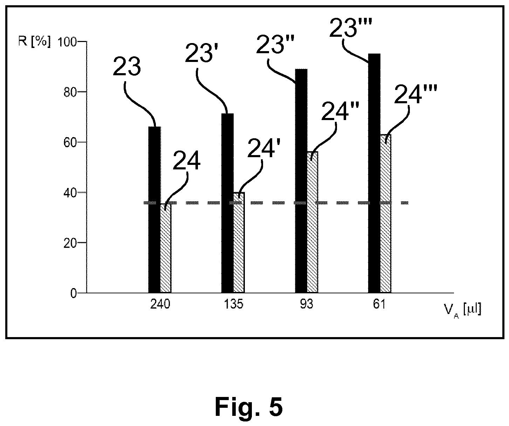

Die Figur 5 zeigt den Einfluss der Volumens *V_{A}* des Akzeptorkanals auf die Wiederfindungsrate R. Die Balken 23, 23`, 23'', 23‴ stellen jeweils die beobachteten Werte für Chlorid dar, während die Balken 24, 24`, 24'', 24‴ Daten für Sulfat repräsentieren. Die dargestellten Werte zeigen jeweils die Wiederfindungsrate R nach 2 min Dialysezeit *t_{D}*. Für alle Messungen wurde ein Donorkanal mit einer Tiefe von 515 µm und einem Volumen *V_{D}* von 240 µl verwendet. Es wurden Akzeptorkanäle mit Volumina *V_{A}* von 240 µl, 135 µl, 93 µl und 61 µl getestet. Es ist zu erkennen, dass die Wiederfindungsrate *R* mit abnehmendem Volumen *V_{A}* des Akzeptorkanals bei gleichbleibender Dialysezeit *t_{D}* höher wird.

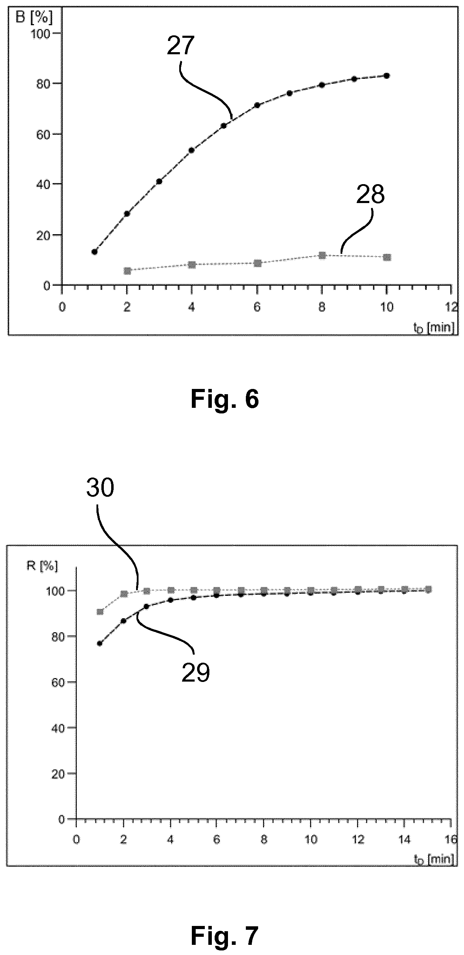

In den Figuren 6 und 7 ist ein Vergleich einer erfindungsgemässen Dialysezelle mit einer solchen aus dem Stand der Technik in Bezug auf den Durchbruch von Matrixmolekülen gezeigt. In diesem Zusammenhang wurde eine Probe mit einer Konzentration von Lignin von 25 mg/l hergestellt. Die Lignin-Konzentration wurde mit einem UV-Detektor bei einer Wellenlänge von 274 nm bestimmt. Der Graph 27 zeigt die Durchbruchsrate B von Lignin als Funktion der Dialysezeit *t_{D}* für eine symmetrische Dialysezelle mit einer Donor- bzw. Akzeptorkanaltiefe von 515 um, einem Donor- bzw. Akzeptorkanalvolumen *V_{D}* bzw. *V_{A}* von 240µl und einer Zelluloseacetat-Membran mit einer Porengrösse von 0.2 µm. Es ist ersichtlich, dass die Durchbruchsrate B nach einer Dialysezeit *t_{D}* von 4 min über 70% liegt. Demgegenüber nimmt, wie in Graph 28 gezeigt, die Durchbruchsrate B für eine asymmetrische Dialysezelle mit einer Donorkanaltiefe von 515µm, einem Donorkanalvolumen *V_{D}* von 240µl, einer Akzeptorkanaltiefe von 515um, einem Akzeptorkanalvolumen *V_{A}* von 90µl und einer Polycarbonat-Membran mit 0.1µm Porengrösse signifikant langsamer über die Dauer der Dialyse zu und erreicht selbst mit 10 min Dialysezeit *t_{D}* nur gerade einen Wert von 10%.

Die Figur 7 zeigt die Wiederfindungsrate R von Nitrat als Funktion der Dialysezeit *t_{D}* für die in Figur 6 gezeigten Dialysekonfigurationen. Graphen 29 und 30 zeigen die Wiederfindungsraten R von Nitrat entsprechend Graphen 27 bzw. 28 in Figur 6. Aus Figur 7 ist ersichtlich, dass der in Figur 6 gezeigte reduzierte Matrix-Durchbruch bei asymmetrischer Dialyse mit feinporiger Membran selbst für den Fall vorliegt, dass die Äquilibrationszeit *t_{A}* bei asymmetrischer Dialyse mit feinporiger Membran geringer ist als bei symmetrischer Dialyse mit grobporiger Membran.

Figuren 6 und 7 zeigen, dass durch die Verwendung einer asymmetrischen Dialysezelle gegenüber einer symmetrischen Dialysezelle durch die Wahl geeigneter Membranen die Äquilibrationszeit *t_{A}* für den Analyten verringert und gleichzeitig der Matrixdurchbruch verringert werden können.

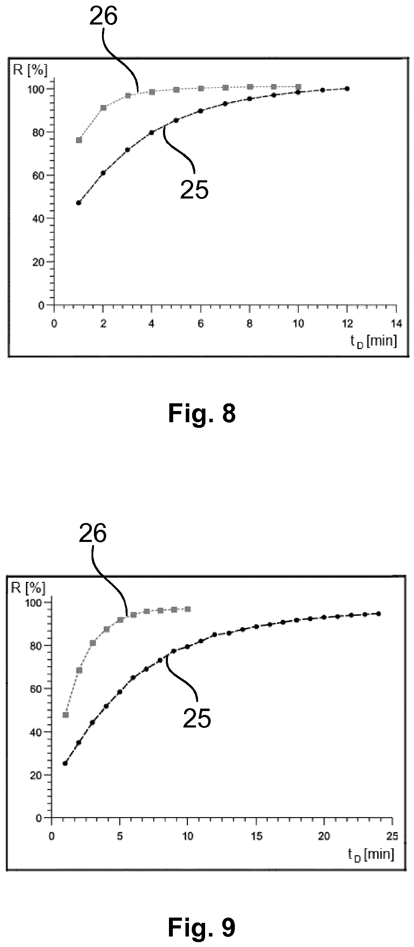

Nachfolgend sowie anhand der Figuren 8 und 9 ist ein Anwendungsbeispiel einer erfindungsgemässen Dialysezelle 1 gegeben. Namentlich wurde der Nitrat- und Sulfatgehalt von Wasserproben aus dem Britzer Kirchteich (Berlin, DE) ermittelt. Dabei handelt es sich um ein Oberflächengewässer, welches Zeichen von Eutrophierung sowie einen hohen Gehalt an Huminstoffen aufweist. Es wurden insgesamt fünf Wasserproben gesammelt und die Konzentration der besagten Anionen ionenchromatographisch bestimmt. Die Probenvorbereitung erfolgte mittels Stopped-Flow-Dialyse.

Es wurden zwei verschiedene Dialysezellen verwendet, welche jeweils einen Aufbau hatten, der dem in der Figur 1 gezeigten entspricht. Bei der ersten Dialysezelle handelte es sich um eine aus dem Stand der Technik bekannte, bei welcher sowohl der Donor- als auch der Akzeptorkanal eine Tiefe von 515 µm und ein Volumen *V*_{A} bzw. *V*_{D} von 240 µl aufwies. Bei der zweiten Dialysezelle handelte es sich um eine erfindungsgemässe, wobei der Donorkanal ebenfalls eine Tiefe von 515 µm und ein Volumen *V*_{D} von 240 µl aufwies. Der Akzeptorkanal hatte eine Tiefe von lediglich 210 µm und ein Volumen *V*_{A} von 90 µl.

In beiden Fällen wurde eine Membran aus Zellulosemischester mit einem Porendurchmesser von 0.05 µm (Merck Millipore) verwendet. Aufgrund der geringen Porengrösse zeichnet sich diese Membran durch eine starke Retention gegenüber potentiell störenden Substanzen, insbesondere makromolekularen Substanzen, wie beispielsweise Huminen oder Ligninen, aus.

Wie anhand der Figuren 8 und 9 gezeigt ist, wurde sowohl für Nitrat als auch für Sulfat erforderliche Dialysezeit *t_{D}* als Vorversuch bestimmt. Hierzu wurden für jedes Ion Standardlösungen mit einer Konzentration von 5 mg/l hergestellt und dialysiert. Die Figur 8 zeigt die Resultate für das Anion Nitrat. Der Graph 25 repräsentiert die Wiederfindungsrate R für einen Akzeptorkanal mit einem Volumen *V*_{A} von 240 µl. Der Graph 26 repräsentiert die entsprechende Wiederfindungsrate R bei einem Akzeptorkanal mit einem Volumen *V*_{A} von 90 µl. Es ist zu erkennen, dass bei der asymmetrischen Dialysezelle für Nitrat bereits mit einer Dialysezeit *t_{D}* von 2 min eine Wiederfindungsrate R von über 90% gegeben ist. Bei der symmetrischen Dialysezelle ist dieser Wert dagegen erst mit einer Dialysezeit *t_{D}* von über 6 min erzielbar. Die Figur 9 zeigt eine analoge Darstellung für das Anion Sulfat. Hier ist zu erkennen, dass eine Wiederfindungsrate R von 90% mit der asymmetrischen Dialysezelle mit ca. 5 min Dialysezeit *t_{D}* erreicht ist, während ein entsprechender Wert beim symmetrischen Zellenaufbau erst mit über 15 min Dialysezeit *t_{D}* erzielt werden kann. Die nachfolgende Tabelle fasst Resultate dieser Vorversuche für Nitrat und Sulfat sowie für weitere Anionen zusammen.

| **Anion** | **Dialyse-zeit *t_{D}* [min]** | **Wiederfindungs-rate R [%]** | **Proben-verbrauch [ml]** | **Dialysezeit *t_{D}* [min]** | **Wiederfindungs-rate R [%]** | **Proben-verbrauch [ml]** |
|---|---|---|---|---|---|---|
| | **240 µl Akzeptorkanal (515 µm Tiefe)** | | | **90 µl Akzeptorkanal (210 µm Tiefe)** | | |
| F⁻ | 15 | 97.2 | 11.6 | 6 | 98.3 | 5.4 |
| Cl⁻ | 9 | 97.1 | 7.5 | 4 | 99.0 | 4.1 |
| NO₂⁻ | 11 | 98.2 | 8.8 | 4 | 98.5 | 4.1 |
| Br⁻ | 9 | 98.4 | 7.5 | 3 | 98.3 | 3.4 |
| **NO₃⁻** | **9** | **97.1** | **7.5** | **3** | **98.8** | **3.4** |
| **SO₄²⁻** | **24** | **94.8** | **16.3** | **8** | **96.4** | **6.8** |

Wie der obigen Tabelle zu entnehmen ist, konnten mit der asymmetrischen Dialysezelle 1 für alle getesteten Anionen kürzere Dialysezeiten *t_{D}* erzielt werden. Die erreichten Wiederfindungsraten *R* lagen im gleichen Bereich wie bei einer symmetrischen Dialysezelle oder waren oft sogar höher. Darüber hinaus konnte mit einem asymmetrischen Aufbau der Dialysezelle grundsätzlich ein geringerer Probenverbrauch erzielt werden.

In der nachfolgenden Tabelle sind die Nitrat- und Sulfatgehalte für die oben genannte Oberflächenwasseranalyse zusammengefasst.

| **Probennummer** | **NO₃⁻** | | **SO₄²⁻** | |
|---|---|---|---|---|
| | **Konzentration [mg/l]** | **RSD [%]** | **Konzentration [mg/l]** | **RSD [%]** |
| 1 | 0.317 | 4.7 | 3.74 | 1.1 |
| 2 | 0.099 | 4.6 | 3.80 | 0.5 |
| 3 | 0.132 | 4.4 | 3.84 | 0.6 |
| 4 | 0.068 | 6.5 | 3.85 | 1.3 |
| 5 | 0.074 | 1.7 | 3.83 | 0.2 |

Neben dem Konzentrationswert für jede einzelne Probe ist darüber hinaus die relative Standardabweichung (RSD) der Konzentration angegeben.

Zusammenfassend ist festzuhalten, dass mit einer erfindungsgemässen Dialysezelle 1 mit asymmetrischem Aufbau deutlich kürzere Dialysezeiten *t_{D}* und damit ein höherer Probendurchsatz erzielt werden kann. Darüber hinaus wurde festgestellt, dass mit einer derartigen Dialysezelle 1 die Menge an benötigter Probe um mindestens einen Faktor 2 reduziert werden kann. Die geringere Zeit, während der die Matrix mit der Dialysemembran 4 in Kontakt ist, reduziert darüber hinaus den unerwünschten Durchbruch von Matrixbestandteilen sowie damit einhergehende negative Auswirkungen auf das Ionenchromatographiesystem.

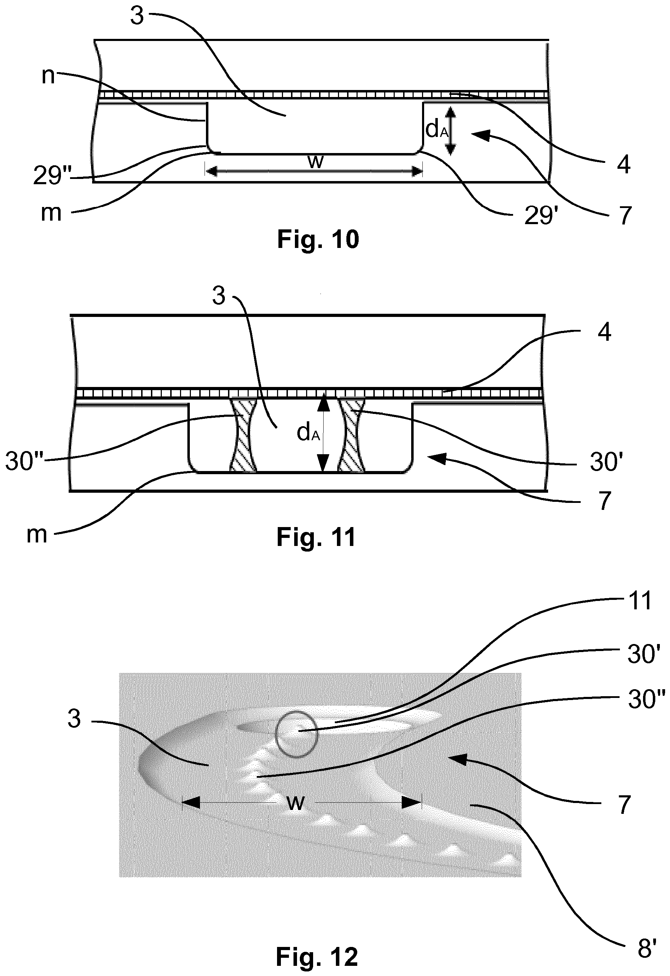

Figur 10 zeigt einen Querschnitt durch eine erfindungsgemässe Akzeptorhalbzelle 7 mit einem Akzeptorkanal 3. Der Querschnitt durch den Akzeptorkanal 7 ist als Rechteck ausgebildet, welches auf der von der Membran abgewandten Seite m abgerundete Ecken 29`, 29" aufweist. Das Verhältnis der Breite w der von der Membran 4 begrenzten Seite zur Tiefe d_{A} des im Querschnitt gezeigten Rechtecks ist nicht massstabsgetreu gezeigt. Das gleiche gilt für den Krümmungsradius r. Erfindungsgemäss ist das Verhältnis der Breite w der von der Membran begrenzten Seite zur Tiefe d_{A} des im Querschnitt gezeigten Rechtecks von 80:1 bis 10:1, besonders bevorzugt von 40:1 bis 15:1 und ganz besonders bevorzugt von 25:1 bis 20:1. Der Krümmungsradius des kreisbogenförmigen Abschnitts, welcher den der Dialysemembran gegenüberliegenden Abschnitt m des Akzeptorkanals 3 mit den an die Dialysemembran anschliessenden, seitlichen Begrenzungen des Akzeptorkanals 3 verbindet, beträgt zwischen 0.05 und 1 mm, bevorzugt zwischen 0.1 und 0.8 mm, ganz besonders bevorzugt zwischen 0.2 und 0.4 mm. Durch die gezeigte Ausgestaltung des Kanals wird das Verhältnis vom Fluidvolumen pro Abschnitt zur Kontaktfläche mit der Membran 4 im gleichen Abschnitt minimiert. Zudem wird verhindert, dass sich beim Strömen des Fluids Akzeptorlösung in den von der Membran 4 abgewandten Ecken 29',29" des Querschnitts ablagert.

Figur 11 zeigt einen Querschnitt durch eine erfindungsgemässe Akzeptorhalbzelle 7 mit einem Akzeptorkanal 3, in welchem mehrere Stützelemente 30', 30" angebracht sind, welche die Membran 4 von der der Membran abgewandten Seite m des Akzeptorkanals 3 beabstanden. Die Stützelemente 30',30" weisen die Höhe d_{A} auf, die der Tiefe des Akzeptorkanals 3 entspricht. Die Stützelemente sind hier als taillierte Zylinder ausgestaltet, um die Fluidströmung so wenig wie möglich zu behindern und gleichzeitig eine möglichst solide Stützung der Membran 4 zu bieten. Durch die Verbreiterung des Umfangs zur Membran hin wird das Risiko vermindert, dass die Membran 4 durch ein konisch zulaufendes Zylinderende oder eine kegelförmige Ausgestaltung des Stützelementes 30', 30" perforiert werden könnte.

Figur 12 zeigt eine perspektivische Ansicht einer erfindungsgemässen Akzeptorhalbzelle 7 mit einem Akzeptorkanal 3. Hier sind die Stützelement kegelförmig konkav gekrümmt ausgebildet. Die Stützelemente 30`, 30" sind einstückig mit dem Akzeptorkanal 3 ausgebildet. Die Stützelemente 30`, 30" sind mittig in der Breite m des Akzeptorkanals 3 angeordnet. Der Abstand zwischen jeweils zwei benachbarten Stützelementen ist über die Kanallänge hinweg konstant. Gezeigt ist ausserdem eine Zuleitung 11 für eine Akzeptorlösung. Das Stützelement 30' ist nahe an der Zuleitung 11 ausgebildet, um ein Aufliegen der Membran 4 (nicht gezeigt) an den Wänden und/oder am Boden des Akzeptorkanals 3 auch und gerade bei der Zuleitungsöffung 11 zu verhindern.

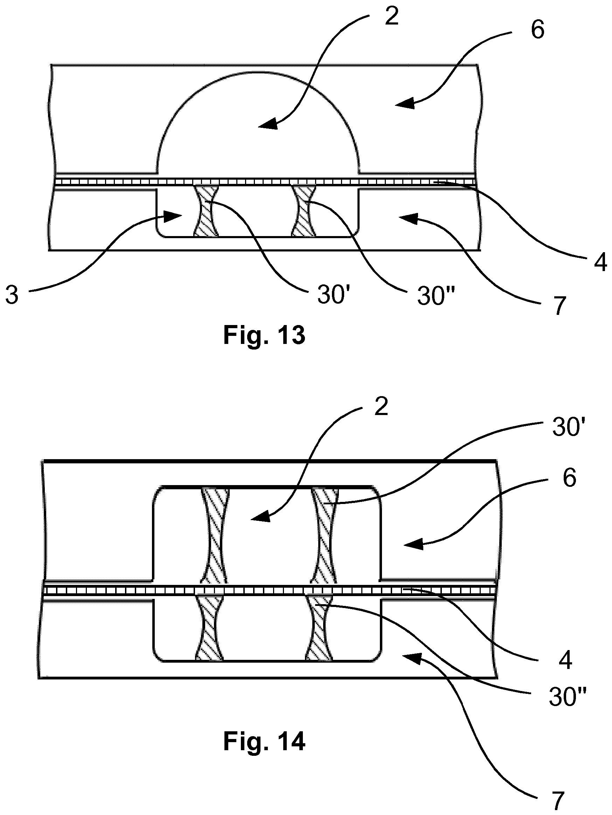

Figuren 13 und 14 zeigen je ein Querschnitt durch eine Anordnungen von jeweils zwei Halbzellen einer erfindungsgemässen Dialysezelle im assemblierten Zustand, jeweils mit Stützelementen 30`, 30". In Figur 13 ist die Donorhalbzelle 6 aufweisend den Donorkanal 2 so ausgebildet, dass der Querschnitt einen Halbkreis zeigt. Die Akzeptorzelle 7 aufweisend den Akzeptorkanal 3 ist so ausgebildet, wie bereits für Fig. 10 bis 12 beschrieben. In Fig. 14 sind Stützelemente 30', 30" sowohl im Donorkanal 2, als auch im Akzeptorkanal 3 ausgebildet. Wenngleich dies nicht bevorzugt ist, so ist es dennoch Teil der Erfindung.

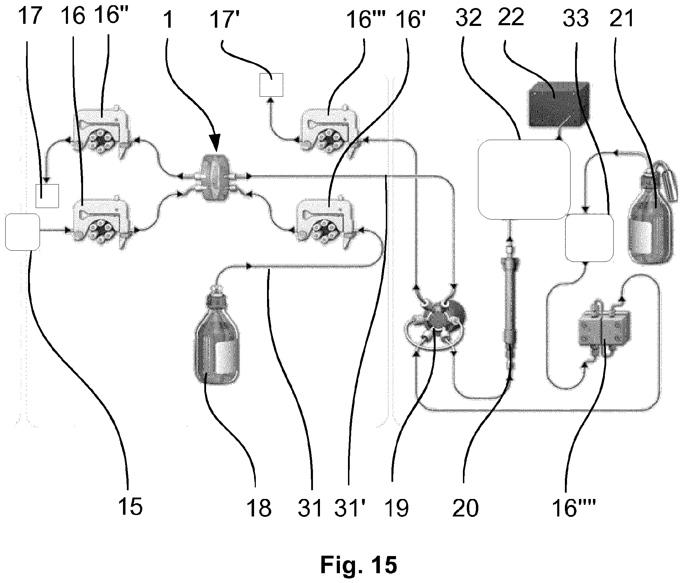

Figur 15 zeigt eine schematische Darstellung einer Vorrichtung zur Probenvorbereitung für ein chemisches Analyseverfahren in einem Dialyseprozess. Wie bereits in Figur 4, umfasst das System eine erfindungsgemässe Dialysezelle 1. Die Donorlösung im Probenbehälter 15 wird mittels einer Pumpe 16 durch die erste Halbzelle der Dialysezelle 1 gepumpt und danach durch die Pumpe 16" zu einem Sammelbehälter 17 geleitet. Die Akzeptorlösung im Akzeptorbehälter 18 wird mittels einer weiteren Pumpe 16' durch die zweite Halbzelle der Dialysezelle 1 gepumpt. Überschüssige Akzeptorlösung wird ebenfalls in einem Sammelbehälter 17' zwecks Entsorgung gesammelt. Die Weiterleitung der Akzeptorlösung zum Sammelbehälter 17' wird zusätzlich durch die Pumpe 16‴ angetrieben.

Das Stopped-Flow-Verfahren entspricht dem zu Figur 4 beschriebenen Verfahren. Zusätzlich ist das System so konfiguriert, dass, während entweder überschüssige Akzeptorlösung dem Sammelbehälter 17' zugeführt wird oder die Akzeptorlösung enthaltend die vorbestimmte Analytkonzentration einem Analyseverfahren zugeführt wird, der Fluss im Donorkreis angehalten wird.

Dadurch wird sichergestellt, dass immer höchstens eine der zwei Halbzellen der Dialysezelle 1 eine Strömung aufweist. Mit anderen Worten werden die Pumpen 16, 16" einerseits und die Pumpen 16' und 16‴ andereseits alternierend betrieben. Während der Dialysezeit *t_{D}* werden ausschliesslich die Pumpen des Donorkreises betrieben und nach Ablauf der Dialysezeit *t_{D}* werden ausschliesslich die Pumpen des Akzeptorkreises betrieben.

Ob die Akzeptorflüssigkeit dem Sammelbehälter 17' oder der Chromatographiesäule 20 zugeführt wird, wird durch Umschalten des Injektionsventils 19 bestimmt. Der eigentliche Chromatographieteil des Chromatographiesystems kann neben dem Eluentenbehälter 21 und der Hochdruckpumpe 16ʺʺ einen Eluent-Entgaser 33 aufweisen. Vor der Detektion im Detektor 22 kann die chromatographisch getrennte Probe wenigstens ein Supressor-Modul 32 durchlaufen.

Wenngleich das nicht gezeigt ist, ist es auch Teil der Erfindung, dass die Pumpe 16 und die Pumpe 16" in einer Zweikanalpumpe, bevorzugt einer peristaltischen Zweikanalpumpe kombiniert sind. Dadurch wird sichergestellt, dass Zufluss und Abfluss der Donorflüssigkeit in/aus der Donorhalbzelle von der gleichen Hubbewegung bestimmt und dadurch synchronisiert sind. Die Pumpen 16' und 16‴ des Akzeptorkreises können zusätzlich oder alternativ als Zweikanalpumpe, insbesondere als peristaltische Zweikanalpumpe, ausgebildet sein, sodass Zufluss und Abfluss der Akzeptorflüssigkeit in/aus der Akzeptorhalbzelle von der gleichen Hubbewegung bestimmt und dadurch synchronisiert sind. Eine solche Ausführungsform verhindert Druckschwankungen in den jeweiligen Halbzellen.

Weiter ist es Teil der Erfindung, dass die Kapillare 31 welche den Akzeptorlösungsbehälter 18 über die Pumpe 16' mit der Akzeptorhalbzelle verbindet, sowie die zweite Kapillare 31', welche die Akzeptorhalbzelle mit dem Injektionsventil 19 verbindet, so ausgestaltet sind, dass der Durchmesser der Kapillare maximal 0.5mm aufweist.

## Patentansprüche

1. Dialysezelle (1) zur Probenvorbereitung für die Ionenchromatographie, umfassend einen Donorkanal (2), einen parallel dazu verlaufenden Akzeptorkanal (3) und eine selektivpermeable Dialysemembran (4), wobei der Donorkanal (2) und der Akzeptorkanal (3) durch die selektivpermeable Dialysemembran (4) voneinander getrennt sind und insbesondere ein in einer Donorlösung im Donorkanal (2) gelöster Analyt (5) durch die Dialysemembran (4) in eine Akzeptorlösung im Akzeptorkanal (3) gelangen kann, wobei der Akzeptorkanal (3) zumindest abschnittsweise ein Volumen *V_{A}* hat, das kleiner ist als ein parallel dazu verlaufendes Volumen *V_{D}* des Donorkanals (2); **dadurch gekennzeichnet dass** die Dialysezelle zwei Halbzellen (6, 7) umfasst, zwischen denen die Dialysemembran (4) angeordnet ist, wobei der Donorkanal (2) und der Akzeptorkanal (3) als jeweils eine Vertiefung in einer Kontaktfläche (8, 8') einer der Halbzellen (6, 7) mit der Dialysemembran (4) ausgebildet sind.

2. Dialysezelle (1) nach Anspruch 1, wobei der Akzeptorkanal (3) auf wenigstens 50%, vorzugsweise wenigstens 70%, bevorzugterweise wenigstens 90%, seiner Länge ein Volumen *V_{A}* hat, das kleiner ist als ein parallel dazu verlaufendes Volumen *V_{D}* des Donorkanals (2) .

3. Dialysezelle (1) nach Anspruch 2, wobei der Akzeptorkanal (3) auf seiner gesamten Länge ein Volumen *V_{A}* hat, das kleiner ist als ein parallel dazu verlaufendes Volumen *V_{D}* des Donorkanals (2).

4. Dialysezelle (1) nach einem der Ansprüche 1 bis 3, wobei die Dialysemembran (4) eine Porengrösse von 0.01 µm bis 1.0 µm, vorzugsweise von 0.02 µm bis 0.5 µm, bevorzugterweise von 0.05 µm bis 0.2 µm, hat.

5. Dialysezelle (1) nach einem der Ansprüche 1 bis 4, wobei der Querschnitt durch den Akzeptorkanal (3) zumindest abschnittweise ein Rechteck ist, welches auf der von der Dialysemembran (4) abgewandten Seite (m) abgerundete Ecken (29', 29") aufweist, wobei das Verhältnis der Breite (w) der von der Dialysemembran (4) begrenzten Seite zur Tiefe (d_{A}) des Querschnitts durch den Akzeptorkanal (3) von 80:1 bis 10:1, bevorzugt von 40:1 bis 15:1 und ganz besonders bevorzugt von 25:1 bis 20:1, beträgt; und/oder wobei die abgerundeten Ecken (29', 29") einen Krümmungsradius von 0.05 bis 1 mm, bevorzugt von 0.1 bis 0.8 mm, ganz besonders bevorzugt von 0.2 bis 0.4 mm aufweisen.

6. Dialysezelle (1) nach einem der Ansprüche 1 bis 5, wobei im Akzeptorkanal (3) wenigstens ein, bevorzugt mehrere Stützelemente (30', 30") ausgebildet sind, welche die Dialysemembran (4) von der der Dialysemembran abgewandten Seite (m) des Akzeptorkanals (3) beabstanden.

7. Vorrichtung zur Probenvorbereitung für ein chemisches Analyseverfahren in einem Dialyseprozess, wobei das chemische Analyseverfahren Chromatographie ist, mit einer Dialysezelle nach einem der Ansprüche 1 bis 6, **dadurch gekennzeichnet dass** der Donorkreis eine erste Pumpvorrichtung (16) aufweist, die die Donorflüssigkeit zur Dialysezelle (1) befördert, und eine zweite Pumpvorrichtung (16") aufweist, die die Donorflüssigkeit von der Dialysezelle (1) wegbefördert, und/oder der Akzeptorkreis eine erste Pumpvorrichtung (16') aufweist, die die Akzeptorflüssigkeit zur Dialysezelle (1) befördert, und eine zweite Pumpvorrichtung (16‴) aufweist, die die Akzeptorflüssigkeit von der Dialysezelle wegbefördert.

8. Vorrichtung nach Anspruch 7, wobei die erste Pumpvorrichtung (16) und die zweite Pumpvorrichtung (16'') des Donorkreises in einer ersten Zweikanalpumpe, bevorzugt einer ersten peristaltischen Zweikanalpumpe, kombiniert sind und/oder die erste Pumpvorrichtung (16') und die zweite Pumpvorrichtung (16‴) des Akzeptorkreises in einer zweiten Zweikanalpumpe, bevorzugt einer zweiten peristaltischen Zweikanalpumpe, kombiniert sind.

9. Vorrichtung nach einem der Ansprüche 7 oder 8, wobei eine erste Kapillare (31), welche einen Akzeptorlösungsbehälter (18) mit einem Akzeptorkanal (3) verbindet und/oder eine zweite Kapillare (31'), welche den Akzeptorkanal (3) mit dem Injektor (19) einer Chromatographievorrichtung verbindet, einen Durchmesser von maximal 0.5 mm, bevorzugt maximal 0.25 mm aufweist/aufweisen.

10. Verwendung einer Dialysezelle (1) nach einem der Ansprüche 1 bis 6 und/oder einer Vorrichtung nach einem der Ansprüche 7 bis 9 zur Probenvorbereitung in einem chemischen Analyseverfahren ausgewählt aus einer Liste bestehend aus Ionenchromatographie (IC) und High Performance Liquid Chromatography (HPLC).

11. Verwendung nach Anspruch 10, umfassend die folgenden Schritte:
- Bereitstellen einer Akzeptorlösung;
- Einleiten einer bestimmten Menge der Akzeptorlösung in den Akzeptorkanal (3) oder in einen den Akzeptorkanal (3) enthaltenden Akzeptorkreis;
- Halten der bestimmten Menge der Akzeptorlösung im Akzeptorkanal (3) oder in dem den Akzeptorkanal (3) enthaltenden Akzeptorkreis;
- Bereitstellen einer zumindest einen Analyten (5) enthaltenden Donorlösung;
- Durchleiten der Donorlösung durch den Donorkanal (2), wodurch der zumindest eine in der Donorlösung enthaltene Analyt (5) durch die Dialysemembran (4) in die Akzeptorlösung gelangt,
wobei mindestens so lange frische Donorlösung durch den Donorkanal (2) geleitet wird, bis die Konzentration des zumindest einen Analyten (5) in der Akzeptorlösung mindestens 90%, vorzugsweise mindestens 95%, bevorzugterweise mindestens 99%, der Konzentration des zumindest einen Analyten (5) in der Donorlösung beträgt.

12. Verwendung nach Anspruch 11, **dadurch gekennzeichnet dass** zu dem Zeitpunkt, in dem die Konzentration des zumindest einen Analyten (5) in der Akzeptorlösung mindestens 90%, vorzugsweise mindestens 95%, bevorzugterweise mindestens 99%, der Konzentration des zumindest einen Analyten (5) in der Donorlösung beträgt, die folgenden Schritte ausgeführt werden:
- Halten der Donorlösung im Donorkanal (2) oder in einem den Donorkanal enthaltenden Donorkreis;
- Ableiten der Akzeptorlösung aus dem Akzeptorkanal (3) und Zuführen der Akzeptorlösung zum Analyseverfahren einer Analysevorrichtung ausgewählt aus einer Liste bestehend aus einer Ionenchromatographievorrichtung (IC) und einer Vorrichtung für High Performance Liquid Chromatography (HPLC).

13. Verwendung nach einem der Ansprüche 11 oder 12, wobei die frische Donorlösung bis zu dem Zeitpunkt, in dem die Konzentration des zumindest einen Analyten (5) in der Akzeptorlösung mindestens 90%, vorzugsweise mindestens 95%, bevorzugterweise mindestens 99%, der Konzentration des zumindest einen Analyten (5) in der Donorlösung beträgt, kontinuierlich durch den Donorkanal (2) geleitet wird.

14. Ionenchromatographiesystem (IC) und/oder System für High Performance Liquid Chromatography (HPLC), umfassend eine Dialysezelle (1) nach einem der Ansprüche 1 bis 6.

## Claims

1. A dialysis cell (1) for sample preparation for ion chromatography, comprising a donor channel (2), an acceptor channel (3) running parallel thereto and a selectively permeable dialysis membrane (4), wherein the donor channel (2) and the acceptor channel (3) are separated from one another by the selectively permeable dialysis membrane (4) and in particular an analyte (5) dissolved in a donor solution in the donor channel (2) can pass through the dialysis membrane (4) into an acceptor solution in the acceptor channel (3), wherein the acceptor channel (3) has, at least sectionally, a volume *V_{A}*, , which is smaller than a volume *V_{D}* of the donor channel (2) running parallel thereto;
**characterized in that** the dialysis cell comprises two half cells (6, 7) between which the dialysis membrane (4) is arranged, the donor channel (2) and the acceptor channel (3) each being formed as an indentation in a contact surface (8, 8') of one of the half cells (6, 7) with the dialysis membrane (4).

2. The dialysis cell (1) according to claim 1, wherein the acceptor channel (3) has on at least 50%, preferably at least 70%, more preferably at least 90%, of its length a volume *V_{A}* which is smaller than a volume *V_{D}* of the donor channel (2) running parallel thereto.

3. The dialysis cell (1) according to claim 2, wherein the acceptor channel (3) has a volume *V_{A}* over its entire length which is smaller than a volume *V_{D}* of the donor channel (2) running parallel thereto.

4. The dialysis cell (1) according to one of claims 1 to 3, wherein the dialysis membrane (4) has a pore size of from 0.01 µm to 1.0 µm, preferably from 0.02 µm to 0.5 µm, more preferably from 0.05 µm to 0.2 µm.

5. The dialysis cell (1) according to one of claims 1 to 4, wherein the cross-section through the acceptor channel (3) is, at least sectionally, a rectangle which has rounded corners (29', 29'') on the side (m) facing away from the dialysis membrane (4), wherein the ratio of the width (w) of the side bounded by the dialysis membrane (4) to the depth (d_{A}) of the cross-section through the acceptor channel (3) is from 80:1 to 10:1, preferably from 40:1 to 15:1 and most preferably from 25:1 to 20:1; and/or wherein the rounded corners (29', 29'') have a radius of curvature of from 0.05 to 1 mm, preferably from 0.1 to 0.8 mm, most preferably from 0.2 to 0.4 mm.

6. The dialysis cell (1) according to one of claims 1 to 5, wherein at least one, preferably several support elements (30', 30'') are formed in the acceptor channel (3), which support elements (30', 30'') space the dialysis membrane (4) from the side (m) of the acceptor channel (3) facing away from the dialysis membrane.

7. A device for sample preparation for a chemical analysis method in a dialysis process, wherein the chemical analysis method is chromatography, with a dialysis cell according to one of claims 1 to 6, **characterized in that** the donor circuit has a first pumping device (16) which conveys the donor liquid to the dialysis cell (1), and a second pumping device (16'') which conveys the donor liquid away from the dialysis cell (1), and/or the acceptor circuit has a first pumping device (16') which conveys the acceptor liquid to the dialysis cell (1), and a second pumping device (16‴) which conveys the acceptor liquid away from the dialysis cell.

8. The device according to claim 7, wherein the first pumping device (16) and the second pumping device (16'') of the donor circuit are combined in a first two-channel pump, preferably a first peristaltic two-channel pump, and/or the first pumping device (16') and the second pumping device (16‴) of the acceptor circuit are combined in a second two-channel pump, preferably a second peristaltic two-channel pump.

9. The device according to one of claims 7 or 8, wherein a first capillary (31), which connects an acceptor solution container (18) to an acceptor channel (3), and/or a second capillary (31'), which connects the acceptor channel (3) to the injector (19) of a chromatography device, has/have a diameter of at most 0.5 mm, preferably at most 0.25 mm.

10. Use of a dialysis cell (1) according to any one of claims 1 to 6 and/or a device according to any one of claims 7 to 9 for sample preparation in a chemical analysis method selected from a list consisting of ion chromatography (IC) and high performance liquid chromatography (HPLC).

11. Use according to claim 10, comprising the following steps:
- providing of an acceptor solution;
- introducing of a certain quantity of the acceptor solution into the acceptor channel (3) or into an acceptor circuit containing the acceptor channel (3);
- holding the specified quantity of the acceptor solution in the acceptor channel (3) or in the acceptor circuit containing the acceptor channel (3);
- providing a donor solution containing at least one analyte (5);
- passing the donor solution through the donor channel (2), whereby the at least one analyte (5) contained in the donor solution passes through the dialysis membrane (4) into the acceptor solution,
wherein fresh donor solution is passed through the donor channel (2) at least until the concentration of the at least one analyte (5) in the acceptor solution is at least 90%, preferably at least 95%, more preferably at least 99%, of the concentration of the at least one analyte (5) in the donor solution.

12. Use according to claim 11, **characterized in that** at the point in time in which the concentration of the at least one analyte (5) in the acceptor solution is at least 90%, preferably at least 95%, more preferably at least 99%, of the concentration of the at least one analyte (5) in the donor solution, the following steps are carried out:
- holding the donor solution in the donor channel (2) or in a donor circuit containing the donor channel;
- discharging the acceptor solution from the acceptor channel (3) and supplying the acceptor solution to the analytical method of an analytical device selected from a list consisting of an ion chromatography device (IC) and a high performance liquid chromatography (HPLC) device.

13. Use according to any one of claims 11 or 12, wherein the fresh donor solution is continuously passed through the donor channel (2) until, at the point in time, the concentration of the at least one analyte (5) in the acceptor solution is at least 90%, preferably at least 95%, more preferably at least 99%, of the concentration of the at least one analyte (5) in the donor solution.

14. Ion chromatography system (IC) and/or system for High Performance Liquid Chromatography (HPLC), comprising a dialysis cell (1) according to any one of claims 1 to 6.

## Revendications

1. Cellule de dialyse (1) pour la préparation d'échantillons pour chromatographie ionique, comprenant un canal donneur (2), un canal accepteur (3) s'étendant parallèlement à celui-ci et une membrane de dialyse (4) à perméabilité sélective, le canal donneur (2) et le canal accepteur (3) étant séparés l'un de l'autre par la membrane de dialyse (4) à perméabilité sélective et, en particulier, un analyte (5) dissous dans une solution donneuse dans le canal donneur (2) pouvant parvenir à traverser la membrane de dialyse (4) vers une solution receveuse dans le canal accepteur (3), le canal accepteur (3) ayant, au moins par sections, un volume *V_{A}* qui est inférieur à un volume *V_{D}* du canal donneur (2) s'étendant parallèlement à celui-ci ;
**caractérisé en ce que** la cellule de dialyse comprend deux demi-cellules (6, 7) entre lesquelles est disposée la membrane de dialyse (4), le canal donneur (2) et le canal accepteur (3) étant réalisés chacun sous la forme d'un creux dans une surface de contact (8, 8') de l'une des demi-cellules (6, 7) avec la membrane de dialyse (4).

2. Cellule de dialyse (1) selon la revendication 1, dans laquelle le canal accepteur (3) a, sur au moins 50%, de préférence au moins 70%, de préférence au moins 90% de sa longueur, un volume *V_{A}* qui est plus petit qu'un volume *V_{D}* du canal donneur (2) qui s'étend parallèlement à celui-ci.

3. Cellule de dialyse (1) selon la revendication 2, dans laquelle le canal accepteur (3) a, sur toute sa longueur, un volume *V_{A}* qui est inférieur à un volume *V_{D}* du canal donneur (2) qui s'étend parallèlement à celui-ci.

4. Cellule de dialyse (1) selon l'une des revendications 1 à 3, dans laquelle la membrane de dialyse (4) a une taille de pores de 0.01 µm à 1.0 µm, de préférence de 0.02 µm à 0.5 µm, de préférence de 0.05 µm à 0.2 µm.

5. Cellule de dialyse (1) selon l'une des revendications 1 à 4, dans laquelle la section transversale à travers le canal accepteur (3) est, au moins par sections, un rectangle qui présente des coins arrondis (29', 29'') sur le côté (m) opposé à la membrane de dialyse (4), la relation de la largeur (w) du côté délimité par la membrane de dialyse (4) à la profondeur (d_{A}) de la section transversale à travers le canal accepteur (3) étant de 80 :1 à 10:1, de préférence de 40:1 à 15:1 et tout particulièrement de 25:1 à 20:1 ; et/ou dans lequel les coins arrondis (29', 29'') ont un rayon de courbure de 0.05 à 1 mm, de préférence de 0,1 à 0,8 mm, de manière particulièrement préférée de 0,2 à 0,4 mm.

6. Cellule de dialyse (1) selon l'une des revendications 1 à 5, dans laquelle au moins un, de préférence plusieurs, éléments de support (30', 30'') sont formés dans le canal accepteur (3), qui écartent la membrane de dialyse (4) du côté (m) du canal accepteur (3) qui est opposé à la membrane de dialyse.

7. Dispositif de préparation d'échantillons pour un procédé d'analyse chimique dans un processus de dialyse, le procédé d'analyse chimique étant la chromatographie, avec une cellule de dialyse selon l'une des revendications 1 à 6, **caractérisé en ce que** le circuit donneur présente un premier dispositif de pompage (16), qui transporte le liquide donneur vers la cellule de dialyse (1), et un deuxième dispositif de pompage (16'') qui transporte le liquide donneur hors de la cellule de dialyse (1), et/ou le circuit accepteur comprend un premier dispositif de pompage (16') qui transporte le liquide accepteur vers la cellule de dialyse (1), et un deuxième dispositif de pompage (16''') qui transporte le liquide accepteur hors de la cellule de dialyse.

8. Dispositif selon la revendication 7, dans lequel le premier dispositif de pompage (16) et le deuxième dispositif de pompage (16'') du circuit donneur sont combinés dans une première pompe à deux canaux, de préférence une première pompe péristaltique à deux canaux, et/ou le premier dispositif de pompage (16') et le deuxième dispositif de pompage (16''') du circuit accepteur sont combinés dans une deuxième pompe à deux canaux, de préférence une deuxième pompe péristaltique à deux canaux.

9. Dispositif selon l'une des revendications 7 ou 8, dans lequel un premier capillaire (31), qui relie un récipient de solution receveuse (18) à un canal accepteur (3), et/ou un deuxième capillaire (31'), qui relie le canal accepteur (3) à l'injecteur (19) d'un dispositif de chromatographie, présente(nt) un diamètre de 0,5 mm maximum, de préférence de 0,25 mm maximum.

10. Utilisation d'une cellule de dialyse (1) selon l'une des revendications 1 à 6 et/ou d'un dispositif selon l'une des revendications 7 à 9 pour la préparation d'échantillons dans un procédé d'analyse chimique choisi dans une liste constituée par la chromatographie ionique (IC) et la chromatographie liquide haute performance (HPLC).

11. Utilisation selon la revendication 10, comprenant les étapes suivantes :
- fournir une solution receveuse;
- introduire une quantité déterminée de la solution receveuse dans le canal accepteur (3) ou dans un circuit accepteur contenant le canal accepteur (3);
- maintenir la quantité déterminée de la solution receveuse dans le canal accepteur (3) ou dans le circuit accepteur contenant le canal accepteur (3);
- fournir une solution donneuse contenant au moins un analyte (5);
- passer la solution donneuse à travers le canal donneur (2), ce qui permet à l'au moins un analyte (5) contenu dans la solution donneuse de passer à travers la membrane de dialyse (4) vers la solution receveuse,
dans lequel on fait passer une solution donneuse fraîche à travers le canal donneur (2) au moins jusqu'à ce que la concentration de l'au moins un analyte (5) dans la solution receveuse représente au moins 90%, de préférence au moins 95%, de préférence au moins 99%, de la concentration de l'au moins un analyte (5) dans la solution donneuse.

12. Utilisation selon la revendication 11, **caractérisée en ce qu'**au moment où la concentration dudit au moins un analyte (5) dans la solution receveuse est d'au moins 90%, de préférence d'au moins 95%, de préférence d'au moins 99%, de la concentration dudit au moins un analyte (5) dans la solution donneuse, les étapes suivantes sont effectuées :
- maintenir la solution donneuse dans le canal donneur (2) ou dans un circuit donneur contenant le canal donneur ;
- évacuer la solution receveuse à partir du canal accepteur (3) et fourniture de la solution receveuse au procédé d'analyse d'un dispositif d'analyse choisi dans une liste constituée d'un dispositif de chromatographie ionique (IC) et d'un dispositif de chromatographie liquide à haute performance (HPLC).

13. Utilisation selon l'une des revendications 11 ou 12, dans laquelle la solution donneuse fraîche est envoyée en continu à travers le canal donneur (2) jusqu'au moment où la concentration dudit au moins un analyte (5) dans la solution receveuse est d'au moins 90%, de préférence d'au moins 95%, de préférence d'au moins 99%, de la concentration dudit au moins un analyte (5) dans la solution donneuse.

14. Système de chromatographie ionique (IC) et/ou système de chromatographie liquide à haute performance (HPLC), comprenant une cellule de dialyse (1) selon l'une quelconque des revendications 1 à 6.
